# EUROPEAN PATENT APPLICATION

(11) **EP 1 808 493 A2**
(43) Date of publication of application: **18.07.2007**
(21) Application number: 06292064.0
(22) Date of filing: 28.12.2006
(51) Int. Cl.: C12Q 1/25, C12Q 1/37, G01N 33/50, G01N 33/68

(54) **Substrates and methods for assaying deubiquitinating enzymes activity**

(30) Priority: 13.01.2006 EP 06290086; 13.01.2006 US 758542 P
(71) Applicant: Hybrigenics S.A., 75014 Paris (FR)
(72) Inventor: Jacq, Xavier, 75012 Paris (FR); Rain, Jean-Christophe, 95120 Ermont (FR)
(74) Representative: Colombet, Alain André

(57) **Abstract**

The invention relates to substrates and methods suitable for assaying deubiquitinating enzyme activity, and in particular for screening inhibitors of enzymes involved in removal of mono or poly-ubiquitin chains from a target protein as well as inhibitors of ubiquitin precursors. Such inhibitors may have utility in the treatment of disease states associated with ubiquitin processing as well as proteolysis.

## Description

The invention relates to substrates and methods suitable for assaying deubiquitinating enzyme activity, and in particular for screening inhibitors of enzymes involved in removal of mono or poly-ubiquitin chains from a target protein as well as inhibitors of ubiquitin precursors. Such inhibitors may have utility in the treatment of disease states associated with ubiquitin processing as well as proteolysis.

Conjugation of ubiquitin and ubiquitin-like proteins to intracellular proteins has emerged as an important mechanism for regulating numerous cellular processes. These include cell cycle progression and signal transduction, transport across the plasma membrane, protein quality control in the endoplasmic reticulum, transcriptional regulation and growth control. The role of ubiquitination in most of these processes is to promote the degradation of specific proteins. A complex enzymatic system is responsible for attaching ubiquitin to and removing it from protein substrates (Pickart CM, Annu. Rev. Biochem. (2001) 70, 503-533; Weissman AM, Nat. Rev. Mol. Cell. Biol. (2001) 2, 169-178).

The role of ubiquitin-dependant proteolysis in the degradation of oncoproteins and tumour suppressors, in cell cycle, in neurodegenerative diseases, in cardio vascular diseases, in stress response and the immune system has become increasingly clear over the last few years (reviewed in Pickart CM, Annu. Rev. Biochem. (2001) 70, 503-533; Glickman MH & Ciechanover A, Physiol. Rev. (2002) 82, 373-428; Nalepa G & Harper WJ, Cancer Treatment Reviews (2003) 29-1, 49-57; Ciechanover A & Brundin P, Neuron (2003) 40, 427-446; Ciechanover A, Biochem. Soc. Trans. (2003) 31-2, 474-481; Hermmann J et al., Cardiovascular Research (2004) 61, 11-21).

Conjugation of ubiquitin to a substrate requires at least three different enzymes. The first enzyme, E1 or ubiquitin-activating enzyme, carries out the ATP-dependant activation of the C-terminus of ubiquitin, forming a covalently bound intermediate with ubiquitin in which the terminal glycine of ubiquitin is linked to the thiol group of a cysteine residue in the E1 active site. Ubiquitin is then transferred to the active site cysteine residue of an ubiquitin-conjugating enzyme or E2. Finally, a third factor, E3 or ubiquitin-protein ligase, catalyzes the transfer of ubiquitin to a lysine residue in the protein substrate (or in cases the N-terminal α-amino group), forming an amide bond. Proteins can be modified on a single or multiple lysine residues by a single ubiquitin or by ubiquitin oligomers. The fate of an ubiquitin-protein conjugate depends in part on the length of the ubiquitin oligomer(s) and on the configuration of ubiquitin-ubiquitin linkages in the ubiquitin chain. Chains of four or more ubiquitins, in which the C-terminus of one ubiquitin is attached to Lysine 48 of the next ubiquitin, efficiently promote binding of the modified protein to the 26S proteasome, with subsequent degradation of the substrate to small peptides but recycling of ubiquitins. In contrast, monoubiquitination or attachment of short lysine 63-linked ubiquitin chains to a protein can have a variety of consequences that do not include proteasomal degradation (Pickart CM, Annu. Rev. Biochem. (2001) 70, 503-533; Amerik AY & Hochstrasser M, Biochimica et Biophysica Acta (2004) 1695, 189-207).

Despite its covalent linkage to many rapidly degraded cellular proteins, ubiquitin itself is a surprisingly long-lived protein *in vivo* (Hicke L, Nat. Rev. Mol. Cell. Biol. (2001) 2, 195-201). This is the result of efficient removal of ubiquitin from its conjugates by deubiquitinating enzymes (DUBs) prior to proteolysis of the conjugated protein. Protein deubiquitination is important for several reasons. When it occurs before the commitment of a substrate to either proteasomal degradation or vacuolar proteolysis, it negatively regulates protein degradation. A proofreading mechanism wherein ubiquitin is removed from proteins inappropriately targeted to the proteasome has also been suggested. Conversely, deubiquitination of proteolytic substrates of the ubiquitin system is necessary for sustaining normal rates of proteolysis by helping to maintain a sufficient pool of free ubiquitin within the cell. Moreover, deubiquitinating enzymes are responsible for processing inactive ubiquitin precursors, and for keeping the 26S proteasome free of the anchored ("free") ubiquitin chains that can compete with ubiquitinated substrates for ubiquitin binding sites (Lam YA et al., Nature (1997) 385, 737-740; Amerik AY & Hochstrasser M, Biochimica et Biophysica Acta (2004) 1695, 189-207).

Deubiquitinating enzymes are part of a large group of enzymes that specifically cleave ubiquitin-linked molecules the terminal carbonyl of the last residue of ubiquitin (Glycine 76). If the ubiquitin-linked molecule is a protein, the linkage is generally an amide bond. Ubiquitin is always synthesized in an active precursor form with a C-terminal extension beyond the terminal ubiquitin glycine. The amide bond that must be hydrolyzed in this case is of the standard peptide variety. When ubiquitin is attached posttranslationally to a protein, it is usually to a lysine ε-amino group, resulting in a distinct amide or "isopeptide" bond. Activated ubiquitin is also susceptible to attack by small intracellular nucleophiles. Some such as glutathione and polyamines, are of considerable abundance, so deubiquitinating enzymes are essential for preventing all of the cellular ubiquitin from being rapidly titrated by these compounds. The precise division of labor between various deubiquitinating enzymes within the cell for cleaving this wide range of ubiquitin conjugates is not well understood. Many deubiquitinating enzymes can hydrolyze different kinds of chemical bonds, although not necessarily with equal efficiency.

The deubiquitinating enzymes fall into five distinct subfamilies based on their sequence similarities and likely mechanism of action. Four of the families are specialized types of cysteine proteases, while the fifth group is a novel type of zinc-dependant metalloprotease. The largest and most diverse group of these subfamilies is the UBP or ubiquitin-specific proteases. The second ubiquitin-specific cysteine protease subfamily is made up of the UCH or ubiquitin carboxy-terminal hydrolases. The third subfamily is made of the OTU (or ovarian-tumor)-related proteases. The fourth family is made up of only one known member, ataxin-3, a protein characterized by a josephin domain. The last subfamily of deubiquitinating enzymes is represented by a subunit of the proteasome, Rpn11/POH1, which has features of a metalloprotease specific for protein-linked ubiquitin (Amerik AY & Hochstrasser M, Biochimica et Biophysica Acta (2004) 1695, 189-207).

*In vivo,* deubiquitinating enzymes remove C-terminal-attached peptides/proteins from ubiquitin. These extensions can be fusion proteins where the α-amino group of the N terminus is bound to the C terminus. In another kind of substrate, the ε-amino group of a lysine residue in a peptide/protein is linked via an isopeptide bond to the C terminus of ubiquitin. These different types of naturally occurring substrates have been used for designing *in vitro* substrates in the past. Naturally occurring fusion proteins such ubiquitin-L40 or polyubiquitin and designed fusion proteins were incubated with deubiquitinating enzymes and the change of the molecular weight was measured upon release of one fusion partner (Layfield R et al., Anal. Biochem. (1999) 274, 40-49). The detection method was based on a separation of products by HPLC or SDS-PAGE. Signal-enhancing systems such as radioactive labelling or epitope mapping have been used to improve the detection limit (Lee JL et al., Biol. Proced. Online (1998) 20, 92-99; Liu CC et al., J. Biol. Chem. (1989) 264, 20333-20338). Substrates containing an isopeptide bond as deubiquitinating enzyme cleavage site are mainly based on short synthetic branched poly-ubiquitin chains (Falquet L et al., FEBS lett. (1995) 359, 73-77; Mason DE et al., Biochemistry (2004) 43, 6535-6544). Polyubiquitin chains can be formed by using the ubiquitin-activating enzyme E1 and the ubiquitin-conjugating enzyme E2-25K. Using the same method, lysine or acetylated lysine has been attached to the C-terminus of ubiquitin (Larsen CN et al., Biochemistry (1998) 37, 3358-3368).

Cleavage of the isopeptide bond was followed by monitoring separation via HPLC. The synthesis of fluorescent isopeptide-linked peptides mimicking the natural substrates has recently been described: fluorescent lysines derivatives were coupled through their e-NH2 terminus group onto the C terminus of ubiquitin by using the ubiquitin-modifying enzymes E1 and E2 (Tirat A et al., Anal. Biochem. (2005) 343, 244-255). The latter substrate has the advantage of mimicking naturally occurring isopeptide linkages but does not fully recapitulate an isopeptide linkage between two ubiquitins in a chain. In another substrate commercially available from several suppliers, 7-amido-4-methylcoumarin (AMC) is linked to the C terminus of ubiquitin (Dang LC et al., Biochemistry (1998) 37, 1868-1879). Deubiquitinating enzymes catalyze the release of AMC from ubiquitin resulting in a fluorescent signal. However, Ubiquitin-AMC has two critical disadvantages when used in screens for deubiquitinating enzyme inhibitors. First, AMC has an excitation wavelength in the UV range. Exciting at 240 to 360 nm is known to excite a significant number of screening compounds and thus will generate a large fraction of false positives. Second, AMC is covalently attached at the C terminal COOH of ubiquitin and not via e-NH2 group as found under physiological conditions. Thus the artificial Ub-AMC substrate might not be optimal for the identification of specific inhibitors of all members of the deubiquitinating enzyme type.

A method or assay which will rapidly and conveniently determine inhibitors of deubiquitinating enzymes will have utility in research and development, particularly in the development of pharmaceutical substances and compositions for the treatment of diseases such as cancer, cardiovascular diseases, neurological disorders, cachexia and muscle wasting. More particularly, such assay would be able to identify those inhibitors which are specifically able to prevent or reduce specific protein degradation. As can be appreciated from the description above, the cellular deubiquitination activities are multiple and involve in many complex pathways and a suitable method for or assay based on those enzymes must overcome a number of problems before rapid and convenient screening for specific inhibitors of deubiquitinating enzymes involved in the pathologies mentioned above.

Artificial substrates containing a peptide bond covalently linking the carboxyl terminus glycine of ubiquitin to "probes" have been described. Examples of such previously described substrates include ubiquitin-AMC or ubiquitin-ethyl ester (Dang LC et al., Biochemistry (1998) 37, 1868-1879; Wilkinson K et al., Biochemistry (1986) 25, 6644-6649). However, these substrates are non-naturally occurring substrates and they cannot be used in high throughput screening methods. Many deubiquitinating enzymes are not able to hydrolyze these artificial substrates.

Moreover, many deubiquitinating enzymes are unable to efficiently process substrates containing a peptide bond covalently linking the carboxyl terminus glycine of ubiquitin to "probes". These are isopeptidase which recognized isopeptide linkage as their natural substrates. Example of such specificities toward poly-ubiquitinated protein substrates targeted to proteasomal degradation includes several published studies (Li M et al, Nature (2002) 416, 648-653; Graner E et al., Cancer cell (2004) 5, 253-261; Kovalenko A et al., (2003) Nature 424, 801-805; Trompouki E et al., Nature (2003) 424, 793-796).

However, ubiquitin chain substrates of defined lengths for deubiquitinating enzymes linked through defined isopeptide bonds and conveniently labelled for high throughput screening purposes have not been described or are not available.

It is the aim of the present invention to deliver a practical method and assay for which inhibitors which are largely specific for deubiquitinating enzymes can be characterized using isopeptide-linked ubiquitin chains of defined length or naturally occurring ubiquitin chains. The use of isopeptide-linked ubiquitin chains of defined length and/or naturally occurring ubiquitin chains makes it possible to discriminate isopeptidases from non-isopeptidase deubiquitinating enzymes.

Furthermore, the proposed substrates for assaying deubiquitinating enzymes make preferably use of Time Resolved Fluorescence (TRF), Fluorescence Resonance Energy Transfer (FRET), or Electrochemoluminescence (ECL) based technologies hence may be used in high throughput screenings.

### Definitions

"Ubiquitin", or "Ub", is a highly conserved 76 amino acid protein expressed in all eukaryotic cells and is best known for its role in targeting proteins for degradation by the 26S proteasome. Preferably, ubiquitin is human ubiquitin. The polypeptide sequence of human ubiquitin, as deposited in database Genpept under accession number P62988, is shown in SEQ ID NO:9. All seven conserved lysines of Ub (K6, 11, 27, 29, 33, 48 and 63) may be used as branching sites for the generation of Ub polymers.

A "deubiquitinating enzyme" denotes a protease which hydrolyses either the ε-linked isopeptide bond or α-linked peptide bond at the C-terminus of ubiquitin, and thereby mediates the removal and processing of ubiquitin from its conjugates (e.g. polyubiquitin chains or chimeric ubiquitin fusion polypeptides). Deubiquitinating enzymes have been reviewed by Nijman et al. (2005, Cell, 123: 773-786).

The term "isopeptidase", as used herein, means a deubiquitinating enzyme having the ability to catalytically cleave the isopeptide bond between the α-carboxyl group of the terminal glycine of ubiquitin (Gly 76) and an ε-amino group in the side chain of a lysine residue of a target protein. Polyubiquitin chains, for instance, are made of ubiquitins linked via isopeptide bonds.

Ribosomes comprise two nonequivalent ribonucleoprotein subunits. The larger subunit (also known as the "large ribosomal subunit") is about twice the size of the smaller subunit (also known as the "small ribosomal subunit"). Both subunits of a ribosome include a RNA, and numerous ribosomal proteins. As used herein, a ribosomal protein denotes a protein from either a small or large ribosomal subunit.

The term "protein tag" denotes a biochemical indicator appended to recombinant expressed proteins. A protein tag may be a fluorescent compound, in particular a fluorescent compound which belongs to a donor/acceptor pair suitable for FRET. The tag may also be a tag which is a binding partner of a ligand/receptor pair, such as hexa histidine-tag (His-tag), biotin, HA (influenza A virus haemagglutinin), Flag, c-myc, DNP (dinitrophenol), GST or Maltose Binding Protein (MBP).

### Deubiquitinating enzymes' substrates

The inventors hypothesized that naturally occurring ubiquitin fusion proteins could be useful tools in order to characterize deubiquitinating enzyme activities. Examples of such previously described substrates include ubiquitin naturally expressed as fusion with ribosomal proteins (Baker RT et al., Nucleic Acids Res. (1991) 19, 1035-1040; Everett RD et al., EMBO J. (1997) 16, 1519-1530). However, no substrate for deubiquitinating enzymes conveniently labelled for high throughput screening purposes has been described or is available.

Accordingly, the invention provides ribosomal protein-linked ubiquitin substrates suitable for identification and characterization of small molecule inhibitors non-isopeptidase deubiquitinating enzymes.

The means for determining the extend of inhibition of deubiquitinating enzymes activity is preferably determined by Time Resolved Fluorescence (TRF), Fluorescence Resonance Energy Transfer (FRET), or Electrochemoluminescence (ECL) based technologies.

Using a gel-based version of the assay described in the present invention as a secondary screen, it is possible to determine whether inhibitors specifically block the deubiquitinating enzyme activity or interfere with the fluorescent assay.

In order to develop an assay for deubiquitinating enzymes based on the FRET phenomena, the inventors had to overcome a certain number of technical difficulties which are not apparent in assays using commercially available substrates:
- synthesize a substrate containing a fusion between ubiquitin and ribosomal proteins. Due to the large size of the substrate (over 14.000 Da), full chemical synthesis of the substrate is not a mean;
- label the two ubiquitin-ribosomal fusion moieties using two different tags;
- select donor/acceptor pairs of fluorescent compounds compatible with substrate recognition by deubiquitinating enzymes; and
- select ligand/receptor pairs suitable with substrate recognition by ligand receptors. For example, anti-ubiquitin antibodies which recognize specifically ubiquitin chains versus free ubiquitin can be used in variations of assay format.

The invention thus provides substrates for deubiquitinating enzymes, in particular non-isopeptidases, which comprise, or consist of the amino acid chain:
R1-ubiquitin-ribosomal protein-R2 in which:
R1 and R2 are protein tags and R1 is different from R2.

The substrates are polypeptide ubiquitin-ribosomal chains which have undergone minor modifications (tagging) which do not disturb the enzyme substrate recognition. More precisely, these substrates can be cleaved by an enzyme having a deubiquitinating activity.

Preferably the substrate includes a naturally occurring ubiquitin precursor.

According to an embodiment, said ubiquitin precursor protein is a mammalian ubiquitin-L40 or ubiquitin-S27 protein. Said ribosomal protein precursor may be for instance human ubiquitin-L40 precursor, which sequence is shown in SEQ ID NO:1 (Swiss-Prot entry NP-001029102), or human ubiquitin-S27, which sequence is shown in SEQ ID NO:2 (Swiss-Prot NP-002945).

R1 and R2 tags may be any tag provided R1 and R2 are different from each other. R1 and R2 are each independently selected from the group consisting of a member of a donor/acceptor pair and a member of a ligand/receptor pair.

R1 and/or R2 may be a member of a donor/acceptor pair of fluorescent compounds for use in FRET or HTRF assays. Examples of suitable donor/acceptor pairs include Europium cryptate/XL665; GFP/YFP; Cy3/Cy5; Fluorescein/Tetramethylrhodamine; CFP/GFP; Dansyl/FITC; Dansyl/Octadecylrhodamine, BFP/DsRFP, IAEDANS/DDPM, Tryptophan/Dansyl, CF/Texas red, Bodipy FUBodipy FL, Rhodamine/Malachite green, FITC/eosin Thiosemicarbazide, B Phycoerythrin/Cy5, and Cy5/C5.5. For other methods as photon, oxygen singlet or electron transfer, donor/acceptor pairs may be chosen by those skilled in the art. Examples include phtalocynine/thioxene derivatives or electron/Ru(bpy)₃²⁺ pairs.

R1 and/or R2 may be a member of a ligand/receptor pair. They may be selected for instance from the group consisting of a hapten, DNP (dinitrophenol), GST (Glutathione S-transferase), biotin, 6HIS (6HIS is a peptide consisting in 6 histidines), c-myc (in which c-myc is a peptide consisting of amino acids 410-419 of the human c-myc protein, EQKLISEEDL, SEQ ID NO:3), FLAG (in which FLAG is a peptide of 8 amino acids (DFKDDDDK (SEQ ID NO:4), DYKAFDNL (SEQ ID NO:5) or DYKDDDDK (SEQ ID NO:6)), and HA (an hemagglutinin epitope consisting of 9 amino acids, YPYDVPDYA, SEQ ID NO:7).

One of R1 and R2 may be a member of a donor/acceptor pair while the other is a member of a ligand receptor pair. Or both R1 and R2 is a member of donor/acceptor pairs or both R1 and R2 is a member of ligand/receptor pairs.

According to a preferred embodiment, said substrate is GST-Ubiquitin-L40-Flag, which sequence is shown in SEQ ID NO:8.

The above substrates, which contain a peptide bond covalently linking the carboxyl terminus glycine of ubiquitin to a ribosomal protein, are efficiently processed by deubiquitinating enzymes of non-isopeptidase type. However, they are likely to be less efficiently processed by isopeptidases. Isopeptidases are deubiquitinating enzymes which recognize isopeptide linkage as their natural substrates.

Accordingly, the invention provides isopeptide-linked ubiquitin chains substrates suitable for identification and characterization of small molecule inhibitor of isopeptidase deubiquitinating enzymes.

To that end, the inventors had to overcome a certain number of technical difficulties which are not apparent in assays using substrates containing a peptide linkage at the carboxy-terminus of glycine 76 of ubiquitin:
- synthesize a substrate containing an isopeptidase linkage between two ubiquitins. Due to the large size of the substrate (over 16.000 Da), full chemical synthesis of the substrate is not a mean;
- label each ubiquitin moieties using two different tags. The enzymes used in the enzymatic synthesis of ubiquitin chains do not easily accommodate tagged version of ubiquitin as a substrate;
- select donor/acceptor pairs of fluorescent compounds compatible with substrate recognition by deubiquitinating enzymes; and
- select ligand/receptor pairs suitable with substrate recognition by ligand receptors. For example, anti-ubiquitin antibodies which recognize specifically ubiquitin chains versus free ubiquitin can be used in variations of assay format.

The invention thus relates to a deubiquitinating enzyme substrate, in particular an isopeptidase substrate, which comprises, or consists of the amino acid chain: 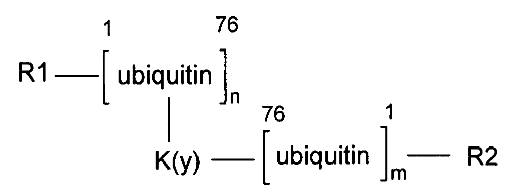 in which:
R1 and R2 are protein tags and R1 is different from R2;
n is an integer which is at least 1;
m is an integer which is at least 1;

Preferably n+m is no more than 4.

K(y) is an isopeptide linkage between a lysine (i.e. any of the seven lysines K6, K11, K27, K29, K33, K48 and K63) of a first ubiquitin and the C-terminal glycine of a second ubiquitin.

When the chain contains more than two ubiquitin monomers, the ubiquitin monomers are linked to each other by isopeptide bonds.

The substrates are poly-ubiquitin chains which have undergone minor modifications (tagging) which do not disturb the enzyme substrate recognition. More precisely, these substrates can be cleaved by an enzyme having a deubiquitinating activity.

Preferably ubiquitin is a mammalian ubiquitin, still preferably human ubiquitin such as shown in SEQ ID NO:9.

Preferably in the above formula n=1 and m=1. According to this embodiment the substrate may be an ubiquitin chain wherein two consecutive ubiquitins are tagged with different protein tags, the consecutive ubiquitins being linked by an isopeptide bond between any of the seven lysines (K6, K11, K27, K29, K33, K48 and K63) of the first ubiquitin and the C-terminal glycine residue (G76) of the second ubiquitin. The substrate may thus consist of the amino acid chain:

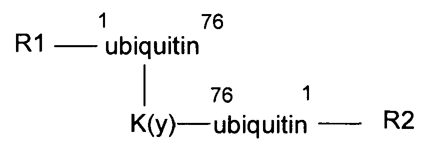

In the deubiquitinating enzyme/isopeptidase substrate of the invention R1 and R2 tags may be any tag provided R1 and R2 are different from each other. R1 and R2 are each independently selected from the group consisting of a member of a donor/acceptor pair and a member of a ligand/receptor pair.

R1 and/or R2 may preferably be a member of a donor/acceptor pair of fluorescent compounds for use in FRET assays. Examples of suitable donor/acceptor pairs include Europium cryptate/XL665, GFP/YFP, Cy3/Cy5; Fluorescein/Tetramethylrhodamine, CFP/GFP, Dansyl/FITC, Dansyl/Octadecylrhodamine, BFP/DsRFP, IAEDANS/DDPM, Tryptophan/Dansyl, CF/Texas red, Bodipy FUBodipy FL, Rhodamine/Malachite green, FITC/eosin Thiosemicarbazide, B Phycoerythrin/Cy5, and Cy5/C5.5. For other methods as photon, oxygen singlet or electron transfer, donor/acceptor pairs may be chosen by those skilled in the art. Examples include phtalocynine/thioxene derivatives or electron/Ru(bpy)₃²⁺ pairs.

R1 and/or R2 may be a member of a ligand/receptor pair. They may be selected for instance from the group consisting of a hapten, DNP, GST, biotin, 6HIS, c-myc, FLAG, and HA.

One of R1 and R2 may be a member of a donor/acceptor pair while the other is a member of a ligand receptor pair. Or both R1 and R2 are a member of donor/acceptor pairs or both R1 and R2 are a member of ligand/receptor pairs.

The invention provides in particular an ubiquitin-dimer substrate which is His-ubiquitin-K48-ubiquitin-biotin, i.e. a substrate which consists of a first ubiquitin, tagged with His, linked to a second ubiquitin, tagged with biotin, via an isopeptide bound between Lys48 of said first ubiquitin and Gly76 of said second ubiquitin.

The invention also provides an ubiquitin-dimer substrate which is Hisubiquitin-K63-ubiquitin-biotin, i.e. a substrate which consists of a first ubiquitin, tagged with His, linked to a second ubiquitin, tagged with biotin, via an isopeptide bound between Lys63 of said first ubiquitin and Gly76 of said second ubiquitin.

### Method of assaying deubiquitinating enzymes activity

A further subject of the present invention is method assaying of deubiquitinating enzyme activity based on measuring a signal resulting from a close proximity transfer between two compounds attached to the enzyme substrate. Therefore no step of separation of the fragments derived from the enzymatic activity is required.

The close proximity assay can be an energy transfer (FRET phenomenon, HTRF® technology, CIS bio international, see, for example, Bazin H et al., Spectrochim. Acta A. Mol. Biomol. Spectrosc. (2001) 57, 2197-2211), a photon transfer, a singlet oxygen transfer (Alphascreen® technology PerkinElmer, see, for example, Beaudet L et al., Genome Res. (2001) 4, 600-608), or an electron transfer (SPA technology, Amersham Biosciences, see for example Udenfriend S et al., Anal. Biochem.(1987) 161-2, 494-500).

The invention relates in particular to an assay for deubiquitinating enzymes activity based on homogenous time-resolved measurement of fluorescence resulting from an energy transfer between a donor fluorescent compound and an acceptor fluorescent compound, which are attached to the substrate. Preferably, this deubiquitinating enzyme has activity of the isopeptidase type.

The FRET (fluorescence resonance energy transfer) phenomenon allows homogenous time-resolved measurement of fluorescence. The use of this technique with rare earth cryptates or chelates, developed in particular by G Mathis et al. (see in particular "Homogenous time-resolved fluorescence energy transfer using rare earth cryptates as a tool for probing molecular interactions in biology", Spectrochimica Acta Part A (2001) 57, 2197-2211) has many advantages which have already allowed several applications in the field of *in vitro* diagnosis and in that of high throughput screening in the pharmaceutical industry.

This method has been use for other proteases (see in particular Preaudat et al, J. of Biomolecular Screening (2002) 7-3, 267-274).

Hence, the invention relates, firstly, to a method of assaying deubiquitinating enzyme activity in a sample, which method comprises the steps consisting of:
a) contacting a substrate which comprises the amino acid chain R1-ubiquitin- ribosomal protein-R2, as defined above, with said sample; and
b) measuring the change in the amount of intact substrate;
wherein a decreased amount of intact substrate is indicative of deubiquitinating activity in the sample.

The invention also relates to a method of assaying deubiquitinating activity in a sample, which method comprises the steps consisting of:
a) contacting a substrate which comprises the amino acid chain 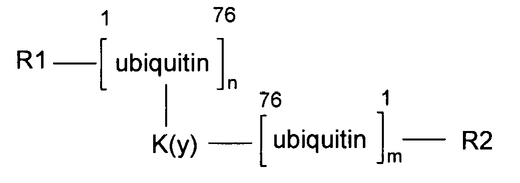 as defined above, with said sample; and
b) measuring the change in the amount of intact substrate;
wherein a decreased amount of intact substrate is indicative of deubiquitinating activity in the sample. In particular said deubiquitinating activity is isopeptidase activity.

In the methods of assaying deubiquitinating enzyme activity according to the invention, said sample may be a deubiquitinating enzyme chosen from recombinant deubiquitinating enzymes, purified deubiquitinating enzymes, non purified deubiquitinating enzymes. In particular, the deubiquitinating enzyme used may be a deubiquitinating enzyme from any type such as a Ubiquitin Specific Protease (USP), a Ubiquitin Carboxy-terminal Hydrolases (UCH), an otubain (OTU); a Jamm/POH1 type of metallo-deubiquitinating enzyme; or any other protein displaying a deubiquitinating activity. Said sample may also be a non-purified source of deubiquitinating enzyme such as a cellular lysate or tissue lysate.

The methods for determining deubiquitinating enzyme activity described above make it possible to study the effects of modulation of these enzymes' activity, exerted by compounds the testing of which is desired.

The expression "modulation of enzyme activity" is intended to mean inhibition or activation of enzyme activity, regardless of the mechanism.

The invention therefore also relates to a method of screening compounds capable of modulating deubiquitinating enzyme activity, comprising the steps consisting of:
a) contacting a substrate which comprises the amino acid chain R1-ubiquitin- ribosomal protein-R2, as defined above, with a deubiquitinating enzyme, in the presence or absence of a test compound,
b) measuring the amount of intact substrate, and
wherein a change in the amount of intact substrate measured in the absence of the test compound compared with that measured in the presence of the test compound is indicative of a compound modulating deubiquitinating activity.

The invention further provides a method of screening compounds capable of modulating deubiquitinating enzyme activity of the isopeptidase type, comprising the steps consisting of:
a) contacting a substrate which comprise the amino acid chain 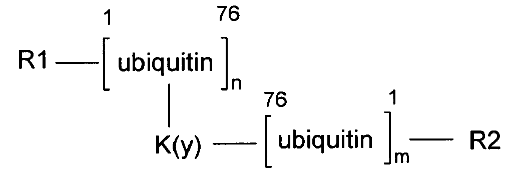 as defined above, with a deubiquitinating enzyme, in the presence or absence of a test compound,
b) measuring the amount of intact substrate, and
wherein a change in the amount of intact substrate measured in the absence of the test compound compared with that measured in the presence of the test compound is indicative of a compound modulating isopeptidase activity.

In particular, in the above methods, an increased amount of intact substrate measured in the absence of the test product compared with that measured in the presence of the test compound is indicative of a compound inhibiting deubiquitinating/isopeptidase activity.

On the contrary, a decreased amount of intact substrate measured in the absence of the test product compared with that measured in the presence of the test compound is indicative of a compound activating deubiquitinating/isopeptidase activity. They may be inhibitors of enzymes involved in removal of mono or poly-ubiquitin chains from a target protein as well as inhibitors of ubiquitin precursors. Such inhibitors may have utility in the treatment of disease states associated with ubiquitin processing as well as proteolysis.

The invention further relates to the inhibitors or activator identified by the method of screening modulators of deubiquitinating enzyme activity, as defined above, as well as pharmaceutical compositions containing these inhibitors.

The deubiquitinating enzyme assayed by the methods of the invention may be a deubiquitinating enzyme chosen from recombinant deubiquitinating enzymes, purified deubiquitinating enzymes, non purified deubiquitinating enzymes. In particular, the deubiquitinating enzyme used may be a deubiquitinating enzyme from any type such as a Ubiquitin Specific Protease (USP), a Ubiquitin Carboxy-terminal Hydrolases (UCH), an otubain (OTU); a Jamm/POH1 type of metallo-deubiquitinating enzyme; or any other protein displaying a deubiquitinating activity.

In the methods of the invention, contacting of the substrate with the sample, or contacting of the substrate and optionally test compound with the deubiquitinating enzyme is carried out in solution, under conditions allowing the substrate to be processed by a deubiquitinating enzyme. More specifically, the solution is a buffer compatible with deubiquitinating enzyme activity. The composition and chemical parameters of the buffer (pH, salinity...) may be readily determined by the one skilled in the art. An example of suitable buffer is 50 mM Tris HCl pH 7.6, Bovine Serum Albumin 0.05%, dithiothreitol (DTT) 5 mM.

The resulting mixture is incubated. Incubation is carried out typically at 37°C. The incubation time may be readily determined by the one skilled in the art. It is generally comprised between 10 to 90 min, or preferably 30 to 60 min.

The substrate may be directly or indirectly labelled with a donor compound and/or with an acceptor compound and the amount of intact substrate is determined by measuring a signal emitted by the acceptor compound, this signal resulting from a transfer, via a close proximity effect, between the donor and the acceptor.

In a particular implementation of this method, the donor compound and the acceptor compound are fluorescent compounds, the close proximity assays is an energy transfer and the signal emitted is a fluorescent signal. Those skilled in the art are able to select the appropriate acceptor fluorescent compound as a function of the donor fluorescent compound chosen. Examples include Europium cryptate/XL665, GFP/YFP, Cy3/Cy5; Fluorescein/Tetramethylrhodamine, CFP/GFP, Dansyl/FITC, Dansyl/Octadecylrhodamine, BFP/DsRFP, IAEDANS/DDPM, Tryptophan/Dansyl, CF/Texas red, Bodipy FUBodipy FL, Rhodamine/Malachite green, FITC/eosin Thiosemicarbazide, B Phycoerythrin/Cy5, and Cy5/C5.5. For other methods as photon, oxygen singlet or electron transfer, donor/acceptor pairs may be chosen by those skilled in the art. Examples include phtalocynine/thioxene derivatives or electron/Ru(bpy)₃²⁺ pairs.

For indirect labelling, the donor and/or acceptor compound is covalently attached to one binding partner of a ligand-receptor pair, the other binding partner of the ligand-receptor pair being covalently attached to the substrate.

The "ligand-receptor pair" denotes two binding partners such as the pairs: hapten/antibody; DNP/anti-DNP antibody in which DNP represents dinitrophenol; GST/anti-GST antibody in which GST represents Glutathione S-transferase; biotin/avidin; 6HIS/anti-6HIS antibody, in which 6HIS is a peptide consisting in 6 histidines; c-myc/anti-c-myc antibody in which c-myc is a peptide consisting of amino acids 410-419 of the human c-myc protein (EQKLISEEDL, SEQ ID NO:3); FLAG/anti-FLAG antibody, in which FLAG is a peptide of 8 amino acids (DFKDDDDK (SEQ ID NO:4), DYKAFDNL (SEQ ID NO:5), or DYKDDDDK (SEQ ID NO:6)); or HA/anti-HA antibody in which HA is an hemagglutinin epitope consisting of 9 amino acids-(YPYDVPDYA, SEQ ID NO:7). Other pairs can be used. These tags/anti-tags pairs are known of those skilled in the art and are commercially available.

Covalent attachment of fluorescent compounds to ubiquitin used for chain synthesis can be generated as previously described (Corsi D et al., J. Biol. Chem. (1995) 270, 8928-8935; Mitsui A et al., Proc Natl. Acad. Sci. (1999) 96, 6054-6059; Beers EP et al., J. Biol. Chem. (1993) 268, 21245-21249).

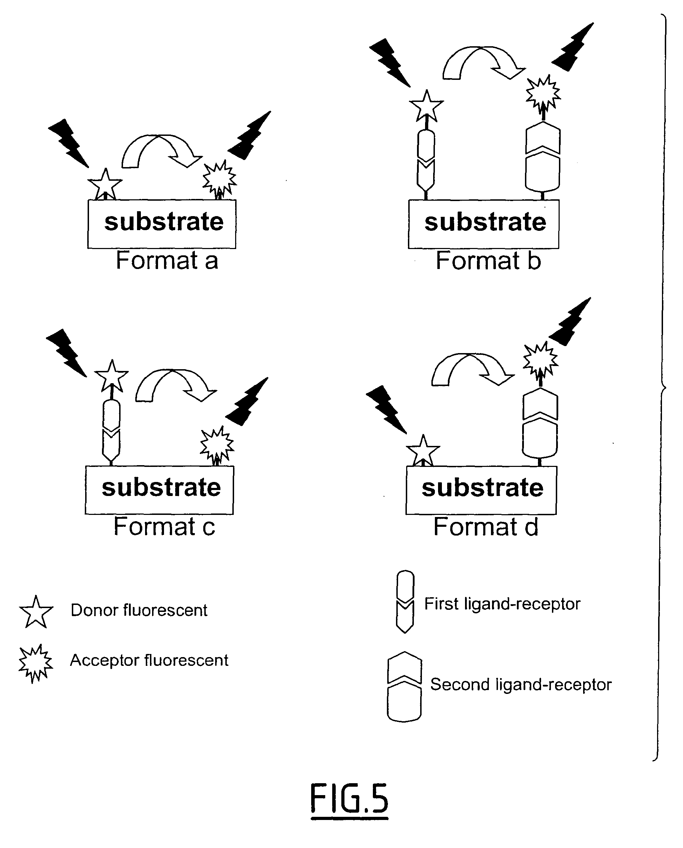

The methods according to the invention can be implemented using various formats. Preferred formats are represented in figure 5.

Where the substrate is indirectly labelled with at least one of the donor compound or the acceptor compound (i.e. at least one of R1 or R2 is a member of a ligand/receptor pair), the step b) of measuring the amount of intact substrate comprises adding to the mixture of substrate and sample, or of substrate and deubiquitinating enzyme and optionally test compound, at the end of incubation time, the other member of the ligand/receptor pair covalently attached to the donor and/or acceptor compound, as appropriate.

In particular where R1 or R2 is GST, for instance, detection of intact substrate may be performed by adding in the reaction mixture an anti-GST antibody covalently linked to either a donor or acceptor compound as appropriate.

The method for detecting a compound capable of modulating enzymatic activity of the deubiquitinating type make it possible to screen libraries of products which can in particular be antibodies, natural products, synthetic products from a library of compounds obtained by combinatorial chemistry, peptides and proteins.

The methods according to the invention have many advantages compared to the method of the prior art, and in particular:
- They are very simple to carry out since it is sufficient to bring the various reagents into contact in order to be able to obtain a fluorescent signal characteristic of enzyme activity of the deubiquitinating type (one step process). No chemical treatment or separation step is required.
- The cost of the method is lower than the previous methods.
- The volume used are very small (20 µl per well or less), which allows the assay to be miniaturized, and makes it possible to save on reagents.
- The incubation time for the reagents are short: as shown in example 1, one hour of incubation is sufficient after the enzyme addition in order to obtain a signal. The method according to the invention makes it possible to rapidly screen libraries of molecules capable of modulating deubiquitinating activity.

The method benefits also from all the advantages known to be associated with the use of Time Resolved Fluorescence in compounds screening.

### Kits for assaying deubiquitinating enzymes activity

Finally, the invention also relates to a kit containing the reagents required to carry out the methods according to the invention, and in particular the following elements:
a) substrate which can be cleaved by an enzyme having an activity of the deubiquitinating type, in particular a substrate which comprise an amino acid chain R1-ubiquitin-Z-ribosomal-protein-R2 or 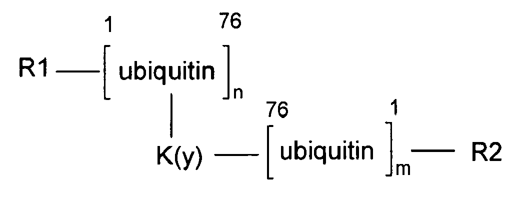 as defined above;
b) a donor fluorescent compound covalently attached or capable of indirectly attaching to said substrate; and
c) an acceptor fluorescent compound covalently attached or capable of indirectly attaching to said substrate.

A donor/acceptor compound capable of indirectly attaching to said substrate is donor/acceptor compound covalently attached to one binding partner of a ligand-receptor pair, the other binding partner of the ligand-receptor pair being covalently attached to the substrate.

The kit may further contain appropriate buffer solutions for carrying out the methods of assaying deubiquitinating enzyme activity as well as a deubiquitinating enzyme as positive control.

The invention will be further illustrated in view of the following figures and examples.

### FIGURES

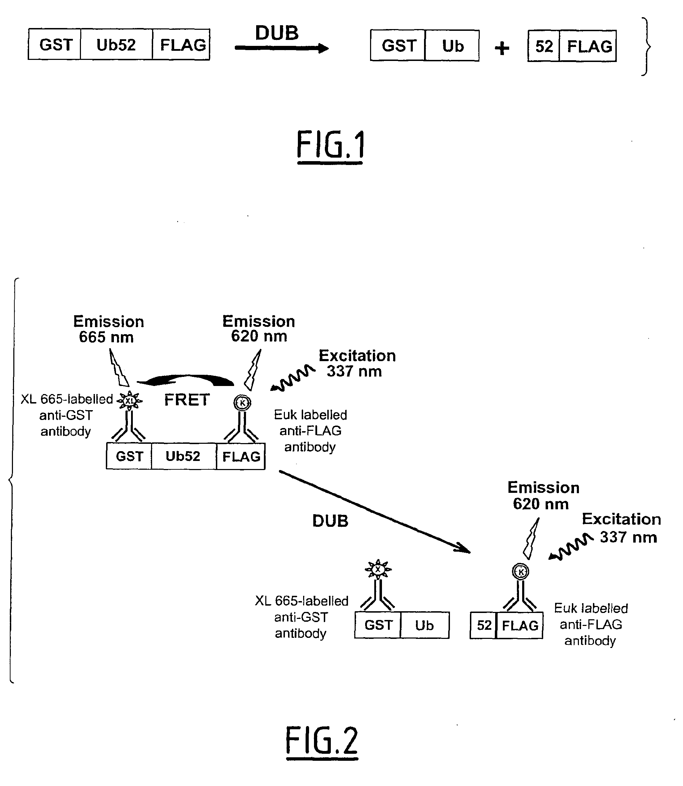

Figure 1 is a schematic representation of ribosomal protein-linked ubiquitin substrates for assaying deubiquitinating enzymes.

Figure 2 is a schematic representation of the method of assaying deubiquitinating enzymes.

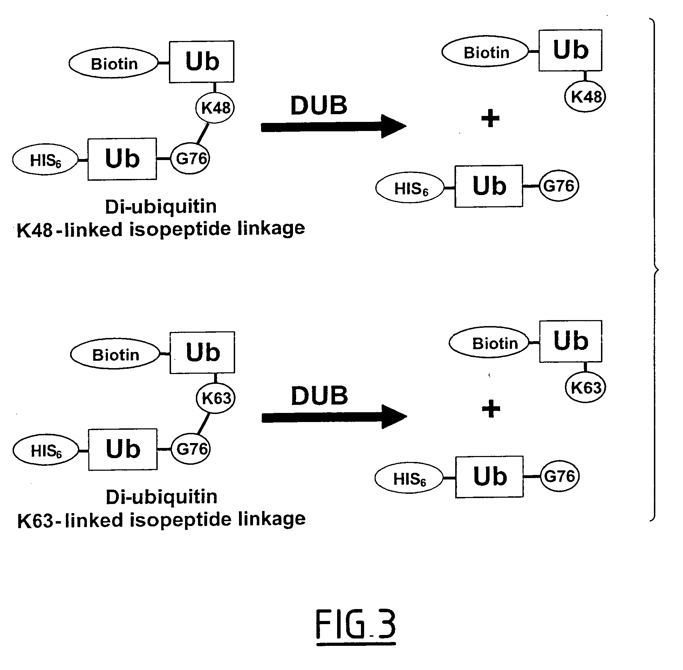

Figure 3 is a schematic representation of isopeptide-linked ubiquitin chains substrates for assaying isopeptidase activity.

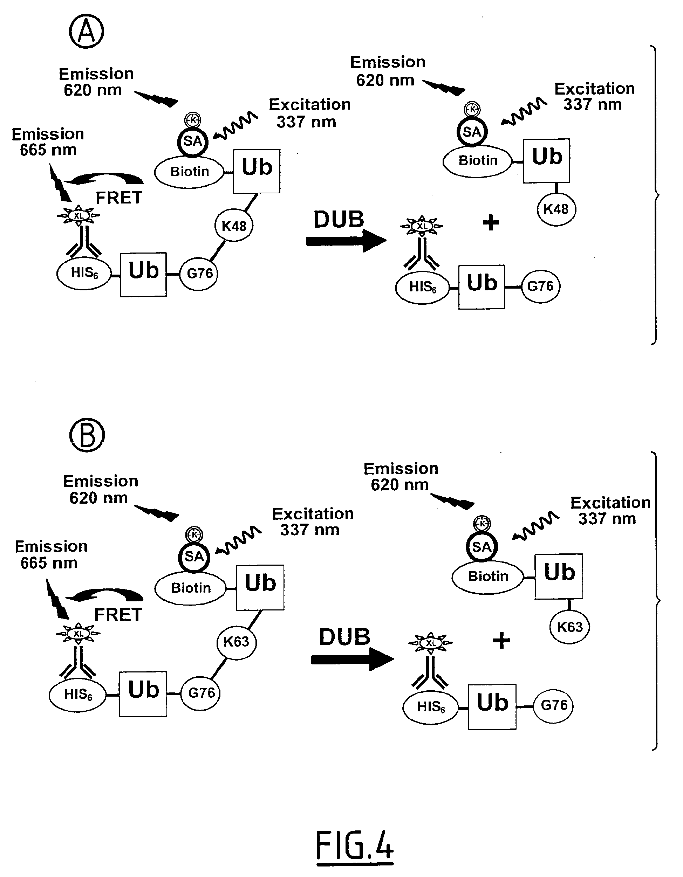

Figure 4 is a schematic representation of the method of assaying isopeptidase activity.

Figure 5 is a schematic representation of the different formats of the method of assaying deubiquitinating/isopeptidase activity. Format a: the substrate can be covalently attached to a donor fluorescent compound and to an acceptor compound. Format b: the substrate is covalently attached to a member of a first ligand-receptor pair and to a member of a second ligand-receptor pair, the donor fluorescent compound is covalently attached to the other member of the ligand-receptor pair and the donor fluorescent compound is attached to the other member of the second ligand-receptor pair. Format c:
the substrate is covalently attached to a donor fluorescent compound and is covalently attached to a member of the ligand-receptor pair, and the acceptor fluorescent compound is covalently attached to the other member of said ligand-receptor pair. Format d: the substrate is covalently attached to the acceptor fluorescent compound and is covalently attached to a member of a ligand-receptor pair, and the donor fluorescent compound is covalently attached to the other member of said ligand-receptor pair.

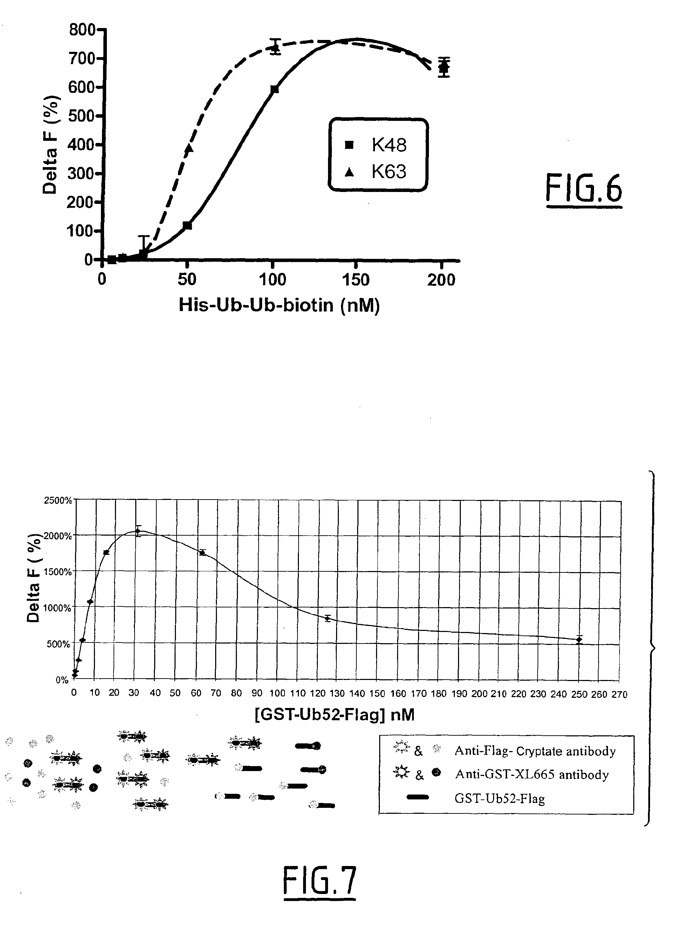

Figure 6 is a graphical representation of the fluorescence emitted by varying concentrations of His-Ub-K48-Ub-biotin or His-Ub-K63-Ub-biotin. Data are expressed as Delta F (%). Delta F = [(Ratio positive - Ratio negative)/Ratio negative] *100. Ratio = Absorbance 665 nm/Absorbance 620 nm.

Figure 7 is a graphical representation which shows the change in fluorescence signal as a function of the concentration in GST-Ub52-Flag. Increasing concentrations of GST-Ub52-Flag were incubated for 60 min at room temperature with anti-Flag-cryptate antibody and anti-GST-XL665 antibody. The dose-dependant increase in HTRF signal is observed below 30 nM GST-Ub-52-Flag. The principle of antibody titration in HTRF experiment is graphically represented at the bottom of the figure.

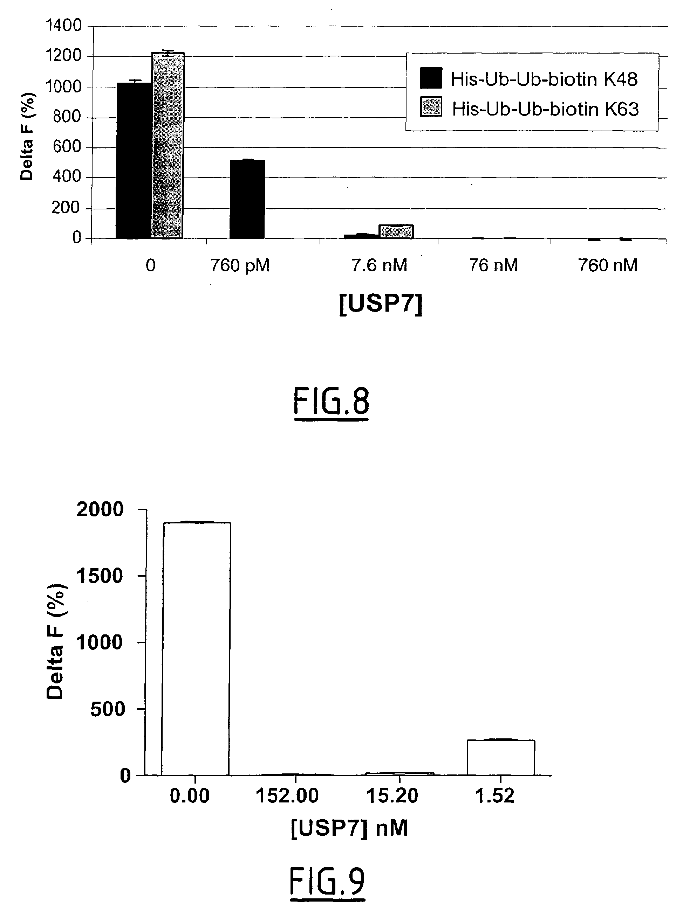

Figure 8 is a graphical representation of changes in the fluorescence signal emitted by His-Ub-K48-Ub-biotin or His-Ub-K63-Ub-biotin with increasing concentrations of USP7 enzyme. Data are expressed as Delta F (%). Delta F = [(Ratio positive - Ratio negative)/Ratio negative] *100. Ratio = Absorbance 665 nm/Absorbance 620 nm.

Figure 9 is a graphical representation of changes in the fluorescence signal emitted by GST-Ub52-Flag with increasing concentrations of USP7 enzyme. Data are expressed as Delta F (%). Delta F = [(Ratio positive - Ratio negative)/Ratio negative] *100. Ratio = Absorbance 665 nm/Absorbance 620 nm.

### EXAMPLES

### Example 1: Preparation of polyubiquitin chains

Ubiquitin chains of various lengths can be obtained also commercially from different sources (Boston Biochem Inc or BioMol).

Because polyubiquitin chains are assembled through isopeptide (versus peptide) bonds, enzymatic synthesis is necessary. Therefore, one's ability to make a given chain presupposes the availability of a conjugating enzyme(s) that is linkage-specific. Description of methods for synthesis of K48- or K63-linked ubiquitin chains of defined lengths have been previously published (for a review, see Raasi S & Pickart CM, Methods in Molecular Biology (2005) 301, 47-55). The procedure involves a series of enzymatic reactions catalyzed by ubiquitin (Ub) conjugation factors that utilize only one of ubiquitin's seven lysine residues as a conjugation site. In each round of synthesis, proximally and distally blocked monoubiquitins (or chains) are joined to produce a doubly blocked chain in high yield. The proximal block consists of an extra C-terminal residue (D77) that is labile to ubiquitin C-terminal enzymes (UCHs). The distal block consists of a cysteine residue (placed at the normal conjugation site) that can be converted to a lysine mimic though alkylation. Successive rounds of deblocking and conjugation can give rise to a chain of any desired length.

Utilisation of two forms of labelled ubiquitin in the chain will result in an ubiquitin chain containing two different tags, thus generating a substrate suitable for the invention herein described.

The ubiquitin chain used in the example is a di-ubiquitin chain consisting in one ubiquitin containing an amino-terminus 6HIS tag and the other ubiquitin labelled at the amino-terminus by biotin, hereinafter referred to as His-Ub-K48-Ub-biotin. A similar chain was also generated but containing a K63 isopeptide linkage instead of the K48 linkage, hereinafter referred to as His-Ub-K63-Ub-biotin. The stock solution of His-Ub-K48-Ub-biotin is 0.5 mg/ml in 50 mM Hepes pH 7.0, 100 mM NaCl, 0.1 mM EDTA. The stock solution of His-Ub-K63-Ub-biotin is 1.0 mg/ml in 50 mM Hepes pH 7.0, 100 mM NaCl, 0.1 mM EDTA.

### Example 2: Assaying the labelled ubiquitin chains using homogenous time-resolved fluorescence (HTRF®) measurement method

The present example makes it possible to validate the use of the labelled ubiquitin chains in an assay based on the time-resolved measurement of fluorescence emitted by radioactive transfer in homogenous medium.

The reagents used were as follows:
- Streptavidin(SA)-europium cryptate conjugate referred to as "SA-K" (CIS bio international), solution at 12 nM in 0.8 M KF (potassium fluoride), 0.05 % Bovine Serum Albumin, Hepes 25 mM pH 7.0.
- Anti-6HIS antibody-XL665 conjugate (CIS bio international) solution at 52 nM in 0.8 M KF, 0.05 % Bovine Serum Albumin, Hepes 25 mM pH 7.0.
- His-Ub-K48-Ub-biotin or His-Ub-K63-Ub-biotin solutions of various concentrations (from 12.5 nM to 400 nM) are prepared by dilutions from the stock solution described above in 50 mM Tris HCl pH 7.6, Bovine Serum Albumin 0.05%, dithiothreitol (DTT) 5 mM.

The assay is carried out on multiwell assay plates. Each well contains 5 µl of SA-K (12 nM), 5 µl of anti-6HIS antibody (52 nM) and 10 µl of His-Ub-K48-Ub-biotin or His-Ub-K63-Ub-biotin of varying concentration. The plates are analyzed on a Pherastar fluorimeter (BMG) after incubation for one hour at ambient temperature (excitation 337 nm, emission 620 and 665 nm) as well as after an overnight incubation at 4°C.

The results obtained after the overnight reading are expressed in figure 6, which shows the change in signal as a function of the concentration in His-Ub-K48-Ub-biotin or His-Ub-K63-Ub-biotin. Figure 6 shows that a signal is obtained for both substrates, which means that an energy transfer clearly takes place between the donor compound (SA-K) and the acceptor compound (anti-6HIS-XL665). The same type of experiments carried out using other compounds (anti-6HIS-K antibody and SA-XL665, for example) resulted in similar albeit less efficient signals. In the range of concentration in which antibodies are not limiting factor for the signal (below 100nM, figure 6), the signal obtained using the present format correlates perfectly with the concentration of His-Ub-K48-Ub-biotin or His-Ub-K63-Ub-biotin, which makes it possible to envision using these products to measure enzyme activity of the deubiquitinating type.

### Example 3: Preparation of ubiquitin-ribosomal protein fusions

A cDNA encoding the fusion protein between ubiquitin and the ribosomal protein L40 (ub52 or uba52 or ubiquitin-L40) was amplified from human RNA using a proprietary human placenta library. The cDNA was subcloned into a bacterial expression vector (pGEX-2T, GE Healthcare), including an additional flag tag at the carboxyl end of the encoded protein. The following primers were used for subcloning in frame with the GST tag the ubiquitin-L40 into pGEX-2T: 5'-cgtggatccatgcagatctttgtgaagaccctc-3' (SEQ ID NO:10) and 5'-gcgaattctttatcgtcatcgtctttgtagtctttgaccttcttcttgggacg-3' (SEQ ID NO:11) into BamHI & EcoRI restriction sites.

For production and purification of recombinant proteins, the plasmid pGEX-2T-Ub52-flag was transformed into E. coli BL21 (Stratagene), grown in LB medium supplemented with 100 mg/ml ampicilin (LB ampi) at 37°C overnight and then diluted 1/100 in LB ampi. The cells were incubated at 37°C until an A600 = 0.6-0.8 was reached. After induction with 0.1 mM isopropyl-β-D-thiogalactopyranoside (IPTG), the culture was incubated at 30°C for 180 min.

Cells were harvested by centrifugation for 15 min at 7000x g at 4°C. Bacterial pellets were lysed in NETN (Tris HCl pH 8.0; EDTA 1 mM; NP40 0.5%; protease inhibitor cocktail, PMSF 1 mM) and briefly sonicated. Insoluble material was removed by centrifugation 30 min at 14000x g. GST-Ub52-flag proteins were purified according to Everett RD et al., EMBO J. (1997) 16, 1519-1530. Briefly, soluble fraction was incubated on Glutathione beads pre-equilibrated in NETN buffer + 0.5% Milk for 120 min at 4°C. Flow Through was recovered. Beads were extensively washed: the last wash was performed in Tris HCl pH 7.6 20 mM; NaCl 100 mM; MgCl₂ 12 mM. Elutions were performed using 20 mM Reduced Glutathione in 50 mM Tris HCl pH 8.0, NaCl 120 mM. All fractions were resolved on a 4-12% NuPAGE following 0.1 M DTT treatment and denaturation and stained with Coomassie Brilliant Blue. Elutions were dialysed over night at 4°C in Tris HCl pH 7.6 20 mM; NaCl 50 mM; DTT 0.5 mM.

### Example 4: Assaying the fusion protein (GST-Ub52-Flag) using homogenous time-resolved fluorescence (HTRF®) measurement method

The present example makes it possible to validate the use of GST-Ub52-Flag in an assay based on the time-resolved measurement of fluorescence emitted by radioactive transfer in homogenous medium.

The reagents used were as follows:
- Anti-flag antibody-europium cryptate conjugate referred to as anti-Flag-K (CIS bio international), solution at 173 nM in 0.8 M KF, 0.1 % Bovine Serum Albumin, Tris HCl 25 mM pH 7.6.
- Anti-GST antibody-XL665 conjugate (CIS bio international), solution at 2.6 µM in 0.8 M KF, 0.1 % Bovine Serum Albumin, Tris HCl 25 mM pH 7.6.
- GST-Ub52-Flag solutions of various concentrations (from 0.5 nM to 250 nM) are prepared by dilutions from the stock solution described above in 50 mM Tris HCl pH 7.6, EDTA 0.5 mM, Bovine Serum Albumin 0.05%, DTT 5 mM.

The assay is carried out on multiwell assay plates. Each well contains 15 µl of a mixture of anti-Flag-K (1.15 nM) and anti-GST-XL665 (17.3 nM) diluted in 0.8 M KF, 0.1 % Bovine Serum Albumin, Tris HCl 25 mM pH 7.6 as well as 5µl of GST-Ub52-Flag substrate of varying concentration. The plates are analyzed on a PHERAstar fluorimeter (BMG) after incubation for one hour at ambient temperature (excitation 337 nm, emission 620 and 665 nm) as well as after an overnight incubation at 4°C.

The results obtained after the overnight reading are expressed in figure 7, which shows the change in signal as a function of the concentration in GST-Ub52-Flag. Figure 7 shows that a signal is obtained which means that an energy transfer clearly takes place between the donor compound (anti-Flag-K) and the acceptor compound (anti-GST-XL665). The same type of experiments carried out using other compounds (anti-GST-K antibody and anti-Flag-XL665, for example) resulted in similar albeit less efficient signals. The signal obtained using the present format correlates perfectly with the concentration of GST-Ub52-Flag, which makes it possible to envision using these products to measure enzyme activity of the deubiquitinating type.

### Example 5: Preparation of a deubiquitinating enzyme

In order to evaluate the activity of deubiquitinating enzymes towards the substrates described above, USP7 (HAUSP) was selected as a typical deubiquitinating enzyme. Several reports had previously characterized the enzymatic activity of USP7 towards isopeptide-linked ubiquitin chains (Meulmeester E et al., Mol. Cell. (2005) 18, 565-576; Van der Knaap JA et al., Mol. Cell. (2005) 17, 695-707; Cummins JM et al., Cell Cycle (2004) 3, 689-692; Li M et al., Mol. Cell. (2004) 879-886; Hu M et al., Cell (2002) 111, 1041-1054).

Full-length USP7 protein (Everett et al., 1997, EMBO J. 16 (3), 566-577; Genpept NP_003461) was expressed in Spodoptera frugiperda (Sf9, Invitrogen) cells using the Bac-to-Bac^{®} Baculovirus system (Invitrogen) according to the manufacturer's instructions. In brief, pFastBac-HT-B-USP7 was transformed into DH10bac cells. Transposition was performed on X-Gal/IPTG agar plates for blue/white selection. Bacmid minipreps were performed using an alkaline lysis procedure. Bacmid minipreps integrity and orientation were verified by PCR using generic and specific primers. Sf9 cells were cultured in InsectXpress medium (Cambrex) at 27°C and are transfected by the fully sequenced bacmid using GeneShuftle 40 (Q-BlOgen). Viruses were recovered in the supernatant at 72 hours post-transfection. Viruses were amplified by infecting Sf9 cells in 50 ml InsectXpress medium in a 150 cm² cell culture flask using 500 µl viral supernatant from the transfected Sf9 cells for 72 hours. Amplified viruses are recovered from supernatants by centrifugation of cell culture media at each amplification round and stored at 4°C. Expression levels in infected Sf9 cells were compared to uninfected cells. Sf9 cells infected for protein production were lysed 72 hours following infection. Cells were washed once in ice cold PBS, resuspended in NP40 lysis buffer (Tris HCl 50 mM pH 7.6; 500 mM NaCl; 0.75% NP40; 10% glycerol; bacterial protease inhibitor cocktail 1/100 (SIGMA); aprotinin 10mg.ml⁻¹ (SIGMA); imidazole 10 mM (Acros); DTT 1 mM)). Cells were vortexed briefly in NP40 lysis buffer and incubated 30 min on ice. Soluble fraction was obtained by centrifugation for 30 min at 14,000 RPM at 4°C.

The fusion proteins were allowed to bind to TALON beads (BD Biosciences, TALON Metal Affinity resin) for 30 min at 4°C under gentle rocking. Beads were extensively washed (with at least 400 bed volume of Wash buffer: Na Phosphate pH 7.0; NaCl 500 mM; Imidazole 10 mM; Triton X-100 0.5%; glycerol 10%). Bound proteins were eluted in Wash Buffer supplemented with 250 mM Imidazole (Sigma). Eluted fractions were resolved on 4-12% NuPAGE. Fractions containing high concentrations of purified proteins were dialyzed (Tris HCl pH 7.6 20 mM; NaCl 50 mM; DTT 0.5 mM) and quantified using Bradford reagent (BioRad).

### Example 6: Assaying the activity of enzymes of the deubiquitinating type with polyubiquitin substrates

The reagents used were as follows:
- Solution of USP7 of various concentrations (3.8 nM; 38 nM; 380 nM; 3.8 µM) in 50 mM Tris HCl pH 7.6, Bovine Serum Albumin 0.05%, DTT 5 mM.
- Streptavidin-europium cryptate conjugate referred to as SA-K (CIS bio international), solution at 12 nM in 0.8 M KF, 0.05 % Bovine Serum Albumin, Hepes 25 mM pH 7.0.
- Anti-6HIS antibody-XL665 conjugate (CIS bio international), solution at 104 nM in 0.8 M KF, 0.05 % Bovine Serum Albumin, Hepes 25 mM pH 7.0.
- His-Ub-K48-Ub-biotin or His-Ub-K63-Ub-biotin solutions at 400 nM are prepared by dilutions from the stock solution described above in 50 mM Tris HCl pH 7.6, Bovine Serum Albumin 0.05%, DTT 5 mM.

The enzyme reaction is carried out by mixing 5 µl of His-Ub-K48-Ubbiotin or 5 µl of His-Ub-K63-Ub-biotin at 400 nM with 5 µl of USP7 solution at varying concentrations (3.8 nM; 38 nM; 380 nM; 3.8 µM). This mixture in incubated for one hour at 37°C on a multiwell assay plate. A 10 µl mixture of 5 µl of SA-K solution (12 nM) plus 5 µl of anti-6HIS-XL antibody (104 nM) is added to each well of the multiwell assay plate. The plate is read after one hour of incubation at room temperature as well as after an overnight incubation at 4°C on a Pherastar fluorimeter (BMG).

The results obtained are expressed in figure 8, which shows the change in the signal for an increase in concentration of enzyme.

The decrease in the signal correlates with the increase in enzyme activity i.e. the cleavage of His-Ub-K48-Ub-biotin and His-Ub-K63-Ub-biotin. The format used is therefore entirely suitable for a method of assaying an enzyme of the deubiquitinating type such as ubiquitin specific protease, but also for determining a modulator of this enzyme activity.

### Example 7: Assaying the activity of enzymes of the deubiquitinating type with ubiquitin-ribosomal protein fusion

The reagents used were as follows:
- Solution of USP7 of various concentrations (1.52 nM; 15.2 nM and 142 nM) in 50 mM Tris HCl pH 7.6, Bovine Serum Albumin 0.05%, DTT 5 mM.
- Anti-Flag-K (CIS bio international), solution at 173 nM in 0.8 M KF, 0.1 % Bovine Serum Albumin, Tris HCl 25 mM pH 7.6.
- Anti-GST antibody-XL665 conjugate (CIS bio international), solution at 2.6 µM in 0.8 M KF, 0.1 % Bovine Serum Albumin, Tris HCl 25 mM pH 7.6.
- GST-Ub52-flag solutions are prepared by dilutions from the stock solution described above in 50 mM Tris HCl pH 7.6, EDTA 0.5 mM, Bovine Serum Albumin 0.05%, DTT 5 mM.

The enzyme reaction is carried out by mixing 5 µl of GST-Ub52-Flag substrate (17.8 nM) with 5 µl of USP7 solution at varying concentrations (1.52 nM; 15.2 nM and 142 nM). This mixture in incubated for one hour at 37°C on a multiwell assay plate. A 10 µl mixture of 5 µl of anti-Flag-K solution (173 nM) plus 5 µl of anti-GST-XL665 antibody (2.6 µM) is added to each well of the multiwell assay plate. The plate is read after one hour of incubation at room temperature as well as after an overnight incubation at 4°C on a PHERAstar fluorimeter (BMG).

The results obtained are expressed in figure 9, which shows the change in the signal for an increase in concentration of enzyme.

The decrease in the signal correlates with the increase in enzyme activity i.e. the cleavage of GST-Ub652-Flag substrate. The format used is therefore entirely suitable for a method of assaying an enzyme of the deubiquitinating type such as ubiquitin specific protease, but also for determining a modulator of this enzyme activity.

### Example 8: Determination of a modulator of enzyme activity of the deubiquitinating type

The same procedures as mentioned above for assaying the activity of enzymes of the deubiquitinating type are carried out but the various reaction mixtures are incubated with identical enzyme concentration, in the presence or absence of a test compound.

The percentage activation or inhibition of the enzyme due to the test compound is determined, by comparison of the results obtained in the presence or absence of the test compound.

## Claims

1. A deubiquitinating enzyme substrate which comprises the amino acid chain :
R1-ubiquitin-ribosomal protein-R2 in which:
R1 and R2 are each independently selected from the group consisting of a member of a donor/acceptor pair and a member of a ligand/receptor pair, and R1 is different from R2.

2. The substrate according to claim 1, wherein said ribosomal protein is S27 or L40 protein.

3. The substrate according to claim 1 or 2, wherein R1 and/or R2 is a member of a donor/acceptor pair selected from the group consisting of Europium cryptate/XL665; GFP/YFP; Cy3/Cy5; Fluorescein/Tetramethylrhodamine; CFP/GFP; Dansyl/FITC; Dansyl/Octadecylrhodamine, BFP/DsRFP, IAEDANS/DDPM, Tryptophan/Dansyl, CF/Texas red, Bodipy FUBodipy FL, Rhodamine 6G/Malachite green, FITC/eosin Thiosemicarbazide, B Phycoerythrin/Cy5, Cy5/C5.5, phtalocynine/thioxene derivatives and electron/Ru(bpy)₃²⁺.

4. The substrate according to any of claims 1 to 3, wherein R1 and/or R2 is a member of a ligand/receptor pair selected from the group consisting of a hapten, DNP (dinitrophenol), GST (Glutathione S-transferase), biotin, 6HIS, c-myc, FLAG and HA.

5. The substrate according to any of claims 1 to 4, which is GST-Ubiquitin-L40-Flag.

6. A deubiquitinating enzyme substrate which comprises the amino acid chain : 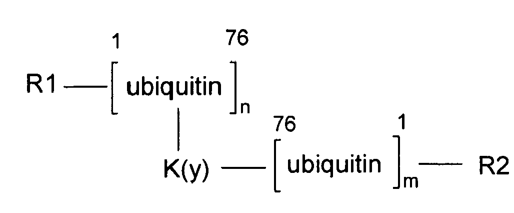 in which:
R1 and R2 are each independently selected from the group consisting of a member of a donor/acceptor pair and a member of a ligand/receptor pair, and R1 is different from R2;
n is an integer which is at least 1;
m is an integer which is at least 1; and
K(y) is an isopeptide linkage between a lysine of first ubiquitin and C-terminal glycine of a second ubiquitin.

7. The substrate according to claim 6 or 7, wherein m+n is no more than 4.

8. The substrate according to claim 6 or 7, wherein n=1 and m= 1.

9. The substrate according to any of claims 6 to 8, wherein R1 and/or R2 is a member of a donor/acceptor pair selected from the group consisting of Europium cryptate/XL665; GFP/YFP; Cy3/Cy5; Fluorescein/Tetramethylrhodamine; CFP/GFP; Dansyl/FITC; Dansyl/Octadecylrhodamine, BFP/DsRFP, IAEDANS/DDPM, Tryptophan/Dansyl, CF/Texas red, Bodipy FUBodipy FL, Rhodamine 6G/Malachite green, FITC/eosin Thiosemicarbazide, B Phycoerythrin/Cy5, Cy5/C5.5, phtalocynine/thioxene derivatives, and electron/Ru(bpy)₃²⁺.

10. The substrate according to any of claims 6 to 9, wherein R1 and/or R2 is a member of a ligand/receptor pair selected from the group consisting of a hapten, DNP (dinitrophenol), GST (Glutathione S-transferase), biotin, 6HIS, c-myc, FLAG and HA.

11. The substrate according to any of claims 6 to 10, which is Hisubiquitin-K48-ubiquitin-biotin or His-ubiquitin-K63-ubiquitin-biotin.

12. A method of assaying deubiquitinating activity in a sample, which method comprises the steps consisting of:
a) contacting a substrate as defined in any of claims 1 to 11, with said sample; and
b) measuring the change in the amount of intact substrate;
wherein the substrate is directly or indirectly labelled with a donor compound and with an acceptor compound and the amount of intact substrate is determined by measuring a signal emitted by the acceptor compound, this signal resulting from a transfer, via a close proximity effect, between the donor and the acceptor,
wherein a decreased amount of intact substrate is indicative of deubiquitinating activity in the sample.

13. The method according to claim 12, wherein said deubiquitinating activity is isopeptidase activity and the substrate is as defined in any of claims 6 to 11.

14. A method of screening compounds capable of modulating deubiquitinating enzyme, comprising the steps consisting of:
a) contacting a substrate as defined in any of claims 1 to 11, with a deubiquitinating enzyme, in the presence or absence of a test compound,
b) measuring the amount of intact substrate, and
wherein the substrate is directly or indirectly labelled with a donor compound and with an acceptor compound and the amount of intact substrate is determined by measuring a signal emitted by the acceptor compound, this signal resulting from a transfer, via a close proximity effect, between the donor and the acceptor,
wherein a change in the amount of intact substrate measured in the absence of the test compound compared with that measured in the presence of the test compound is indicative of a compound modulating deubiquitinating activity.

15. The method according to claim 14, wherein said deubiquitinating activity is isopeptidase activity and the substrate is as defined in any of claims 6 to 11.

16. The method according to claim 14 or 15, wherein an increased amount of intact substrate measured in the absence of the test product compared with that measured in the presence of the test compound is indicative of a compound inhibiting deubiquitinating activity.

17. The method according to claim 14 or 15, wherein a decreased amount of intact substrate measured in the absence of the test product compared with that measured in the presence of the test compound is indicative of a compound activating deubiquitinating activity.

18. The method according to any of claims 12 to 17, wherein said donor and acceptor compounds are fluorescent compounds.

19. A kit for assaying deubiquitinating activity and/or screening compounds capable of modulating deubiquitinating activity which comprises:
a) a substrate as defined in any of claims 1 to 11;
b) a donor fluorescent compound covalently attached or capable of indirectly attaching to said substrate; and
c) an acceptor fluorescent compound covalently attached or capable of indirectly attaching to said substrate.


SEQUENCE LISTING

<110>  Hybrigenics

<120>  Substrates and methods of assaying deubiquitinating enzymes

<130>  BET 06P1417

<160>  11

<170>  PatentIn version 3.3

<210>  1
<211>  128
<212>  PRT
<213>  Homo sapiens

<220>
<221>  MISC_FEATURE
<222>  (1)..(128)
<223>  ubiquitin and ribosomal protein L40 precursor

<400>  1

Met Gln Ile Phe Val Lys Thr Leu Thr Gly Lys Thr Ile Thr Leu Glu
1               5                   10                  15

Val Glu Pro Ser Asp Thr Ile Glu Asn Val Lys Ala Lys Ile Gln Asp
            20                  25                  30

Lys Glu Gly Ile Pro Pro Asp Gln Gln Arg Leu Ile Phe Ala Gly Lys
        35                  40                  45

Gln Leu Glu Asp Gly Arg Thr Leu Ser Asp Tyr Asn Ile Gln Lys Glu
    50                  55                  60

Ser Thr Leu His Leu Val Leu Arg Leu Arg Gly Gly Ile Ile Glu Pro
65                  70                  75                  80

Ser Leu Arg Gln Leu Ala Gln Lys Tyr Asn Cys Asp Lys Met Ile Cys
                85                  90                  95

Arg Lys Cys Tyr Ala Arg Leu His Pro Arg Ala Val Asn Cys Arg Lys
            100                 105                 110

Lys Lys Cys Gly His Thr Asn Asn Leu Arg Pro Lys Lys Lys Val Lys
        115                 120                 125

<210>  2
<211>  156
<212>  PRT
<213>  Homo sapiens

<220>
<221> MISC_FEATURE
<222> (1)..(156)
<223> ubiquitin and ribosomal protein S27a precursor

<400> 2

Met Gln Ile Phe Val Lys Thr Leu Thr Gly Lys Thr Ile Thr Leu Glu
1               5                   10                  15

Val Glu Pro Ser Asp Thr Ile Glu Asn Val Lys Ala Lys Ile Gln Asp
            20                  25                  30

Lys Glu Gly Ile Pro Pro Asp Gln Gln Arg Leu Ile Phe Ala Gly Lys
        35                  40                  45

Gln Leu Glu Asp Gly Arg Thr Leu Ser Asp Tyr Asn Ile Gln Lys Glu
    50                  55                  60

Ser Thr Leu His Leu Val Leu Arg Leu Arg Gly Gly Ala Lys Lys Arg
65                  70                  75                  80

Lys Lys Lys Ser Tyr Thr Thr Pro Lys Lys Asn Lys His Lys Arg Lys
                85                  90                  95

Lys Val Lys Leu Ala Val Leu Lys Tyr Tyr Lys Val Asp Glu Asn Gly
            100                 105                 110

Lys Ile Ser Arg Leu Arg Arg Glu Cys Pro Ser Asp Glu Cys Gly Ala
        115                 120                 125

Gly Val Phe Met Ala Ser His Phe Asp Arg His Tyr Cys Gly Lys Cys
    130                 135                 140

Cys Leu Thr Tyr Cys Phe Asn Lys Pro Glu Asp Lys
145                 150                 155


<210> 3
<211> 10
<212> PRT
<213> Artificial

<220>
<223> amino acids 410-419 of the human c-myc protein

<400> 3

Glu Gln Lys Leu Ile Ser Glu Glu Asp Leu
1               5                   10

<210>  4
<211>  8
<212>  PRT
<213>  Artificial

<220>
<223>  FLAG

<400>  4

Asp Phe Lys Asp Asp Asp Asp Lys
1               5


<210>  5
<211>  8
<212>  PRT
<213>  Artificial

<220>
<223>  FLAG

<400>  5

Asp Tyr Lys Ala Phe Asp Asn Leu
1               5


<210>  6
<211>  8
<212>  PRT
<213>  Artificial

<220>
<223>  FLAG

<400>  6

Asp Tyr Lys Asp Asp Asp Asp Lys
1               5


<210>  7
<211>  9
<212>  PRT
<213>  Artificial

<220>
<223>  HA epitope

<400>  7

Tyr Pro Tyr Asp Val Pro Asp Tyr Ala
1               5


<210>  8
<211>  361
<212>  PRT

<213>  Artificial

<220>
<223>  GST-Ubiquitin-L40-Flag substrate

<400>  8

Met Ser Pro Ile Leu Gly Tyr Trp Lys Ile Lys Gly Leu Val Gln Pro
1               5                   10                  15

Thr Arg Leu Leu Leu Glu Tyr Leu Glu Glu Lys Tyr Glu Glu His Leu
            20                  25                  30

Tyr Glu Arg Asp Glu Gly Asp Lys Trp Arg Asn Lys Lys Phe Glu Leu
        35                  40                  45

Gly Leu Glu Phe Pro Asn Leu Pro Tyr Tyr Ile Asp Gly Asp Val Lys
    50                  55                  60

Leu Thr Gln Ser Met Ala Ile Ile Arg Tyr Ile Ala Asp Lys His Asn
65                  70                  75                  80

Met Leu Gly Gly Cys Pro Lys Glu Arg Ala Glu Ile Ser Met Leu Glu
                85                  90                  95

Gly Ala Val Leu Asp Ile Arg Tyr Gly Val Ser Arg Ile Ala Tyr Ser
            100                 105                 110

Lys Asp Phe Glu Thr Leu Lys Val Asp Phe Leu Ser Lys Leu Pro Glu
        115                 120                 125

Met Leu Lys Met Phe Glu Asp Arg Leu Cys His Lys Thr Tyr Leu Asn
    130                 135                 140

Gly Asp His Val Thr His Pro Asp Phe Met Leu Tyr Asp Ala Leu Asp
145                 150                 155                 160

Val Val Leu Tyr Met Asp Pro Met Cys Leu Asp Ala Phe Pro Lys Leu
                165                 170                 175

Val Cys Phe Lys Lys Arg Ile Glu Ala Ile Pro Gln Ile Asp Lys Tyr
            180                 185                 190

Leu Lys Ser Ser Lys Tyr Ile Ala Trp Pro Leu Gln Gly Trp Gln Ala
        195                 200                 205

Thr Phe Gly Gly Gly Asp His Pro Pro Lys Ser Asp Leu Val Pro Arg
    210                 215                 220

Gly Ser Gln Ile Phe Val Lys Thr Leu Thr Gly Lys Thr Ile Thr Leu
225                 230                 235                 240

Glu Val Glu Pro Ser Asp Thr Ile Glu Asn Val Lys Ala Lys Ile Gln
                245                 250                 255

Asp Lys Glu Gly Ile Pro Pro Asp Gln Gln Arg Leu Ile Phe Ala Gly
            260                 265                 270

Lys Gln Leu Glu Asp Gly Arg Thr Leu Ser Asp Tyr Asn Ile Gln Lys
        275                 280                 285

Glu Ser Thr Leu His Leu Val Leu Arg Leu Arg Gly Gly Ile Ile Glu
    290                 295                 300

Pro Ser Leu Arg Gln Leu Ala Gln Lys Tyr Asn Cys Asp Lys Met Ile
305                 310                 315                 320

Cys Arg Lys Cys Tyr Ala Arg Leu His Pro Arg Ala Val Asn Cys Arg
                325                 330                 335

Lys Lys Lys Cys Gly His Thr Asn Asn Leu Arg Pro Lys Lys Lys Val
            340                 345                 350

Lys Asp Tyr Lys Asp Asp Asp Asp Lys
        355                 360

<210>  9
<211>  76
<212>  PRT
<213>  Homo sapiens

<220>
<221>  MISC_FEATURE
<222>  (1)..(76)
<223>  ubiquitin

<400>  9

<210>  10
<211>  33
<212>  DNA
<213>  Artificial

<220>
<223>  primer

<400>  10
cgtggatcca tgcagatctt tgtgaagacc ctc                                    33

<210>  11
<211>  53
<212>  DNA
<213>  Artificial

<220>
<223>  primer

<400>  11
gcgaattctt tatcgtcatc gtctttgtag tctttgacct tcttcttggg acg              53